# EUROPEAN PATENT SPECIFICATION

(11) **EP 1 289 512 B1**
(45) Date of publication and mention of the grant of the patent: **09.03.2005**
(21) Application number: 01937712.6
(22) Date of filing: 23.05.2001
(51) Int. Cl.: A61K 31/09, A61P 25/28, A61P 43/00

(54) **AGENTS AND METHODS FOR INCREASING BRAIN CHAPERONIN LEVELS**
MITTEL UND VERFAHREN ZUR ERHÖHUNG DES CHAPERONIN-SPIEGELS IM GEHIRN
AGENTS ET METHODES D'AUGMENTATION DES NIVEAUX DE CHAPERONINES CEREBRALES

(30) Priority: 24.05.2000 US 206854 P
(43) Date of publication of application: 12.03.2003
(73) Proprietor: Holtzman, Jordan L., Minneapolis, MN 55409 (US)
(72) Inventor: Holtzman, Jordan L., Minneapolis, MN 55409 (US)
(74) Representative: Plougmann & Vingtoft a/s
(86) International application number: PCT/US2001/016914
(87) International publication number: WO 2001/089500

(56) References cited:
- WO-A-00/42990
- WO-A-99/29657
- US-A- 2 535 000
- MORRELL, S.L., ET AL.: "Effects of methoxychlor administration to male rats on hepatic microsomal iodothyronine 5'-deiodinase, form 1." JOURNAL OF PHARMACOLOGY AND EXPERIMENTAL THERAPEUTICS, vol. 294, no. 1, 2000, pages 308-312, XP001031098
- MORRELL, S.L. ET AL: "Iodothryronine 5'-deiodinase (ID-1) inhibition by chronic methoxychlor administration" JOURNAL OF INVESTIGATIVE MEDICINE, vol. 45, no. 7, - 1997 page 345A XP002187122
- DODGE, J.A., ET AL.: "Environmental estrogens: Effects on cholesterol lowering and bone in the ovariectomized rat." JOURNAL OF STEROID BIOCHEMISTRY AND MOLECULAR BIOLOGY, vol. 59, no. 2, - 1996 pages 155-161, XP001042355
- DATABASE WPI Section Ch, Week 198525 Derwent Publications Ltd., London, GB; Class C03, AN 1985-146747 XP002186753 & CA 1 187 409 A (CANADA MIN ENVIRONMENT), 21 May 1985 (1985-05-21)
- HOLTZMAN, J. L. ET AL.: "A chaperon binds to B-Amyloids: Implications for the etiology of Alzheimer's disease." MOLECULAR BIOLOGY OF THE CELL, vol. 9, no. SUPPL, 1998, page 106A XP001042172
- SOTO, C. : "B-Amyloid disrupting drugs. Potential in the treatment of Alzheimer's disease" CNS DRUGS, vol. 12, no. 5, 1999, pages 347-356, XP001042155
- TAGUCHI, J. ET AL.: "Differential alteration of calreticulin and immunoglobulin binding proteins (BIP) in cerebral cortex of alzheimer's disease brain" SOCIETY FOR NEUROSCIENCE ABSTRACTS, vol. 25, no. 1-2, 1999, page 591 XP001042171

## Description

This application is being filed as a PCT application by JORDAN LOYAL HOLTZMAN, a United States national and resident, designating all countries.

This application claims priority to United State Provisional Application Serial No. 60/206,854, filed on May 24, 2000, entitled AGENTS AND METHODS FOR INCREASING BRAIN CHAPERONIN LEVELS.

### Background of the Invention

Several models have been advanced to explain age-related physiological problems, such as loss in muscle mass, failure of the immune system, and decreases in cognitive skills. Such models include, for example, increased programmed cell death, i.e. apoptosis; accumulation of oxidant damage; failure of the cell to maintain the telomeres at the ends of the chromosomes; and defects in responding to stress. The observed age-related defects in responding to stress may involve chaperone proteins.

At the cellular level the most important stress proteins are the chaperones. Chaperones play an important role in cellular function. They help to realign proteins into their native state, thereby renaturing damaged proteins and aid the final steps of protein folding by directing newly synthesized proteins into their final, optimal structure. Chaperones also help stabilize the final protein product, such as by the formation of intra- and intermolecular disulfide bonds. One such family of chaperones is known as thiol:protein disulfide oxidoreductases (TPDOs). Studies of the stress proteins and chaperones support the concept that many of the age-related functional declines are associated with decreases in the activity of the chaperone systems. Decreased levels and activity of chaperones can result in increased formation of improperly folded and insoluble masses of proteins.

Insoluble masses, or plaques, of the β-amyloid protein in the brain are found in Alzheimer's disease patients. When the β-amyloid precursor is inserted into the plasma membrane β-amyloid protein is cleaved off of the precursor protein. β-amyloid readily aggregates in physiological solutions forming plaques. This aggregation is not observed for the cerebrospinal fluid of a non-Alzheimer's patient. This indicates that normal cerebrospinal fluid contains a component that prevents β-amyloid aggregation and thereby the formation of plaques associated with Alzheimer's disease. In an effort to determine the identity of this component, which is associated with Alzheimer's disease, a β-amyloid chaperone protein complex was isolated. This complex was analyzed to determine the identity of the chaperone. It was discovered that the chaperone protein that was complexing with the β-amyloid was ERP57. The levels of ERP57 play a role in Alzheimer's disease and other related diseases. This is described in PCT Application No. 99/25593.

The Balandrin et al. reference (PCT/US98/26315) discloses agents that modulate CNS activity, by enhancing inhibitory, or decreasing excitatory, neurotransmission. See page 10, paragraph 5. The reference discloses a list of neurological disorders or diseases for which such agents may be used including spasticity, skeletal muscle spasms, restless leg syndrome, anxiety, stress, multiple sclerosis, stroke, head trauma, spinal cord injuries, Parkinson's, Huntington's, Alzheimer's, amyotrophic lateral sclerosis, migraine headaches, and bipolar disorder. See page 8, paragraph 3. However, closely related compounds have known toxicity problems.

### Summary of the Invention

The present invention generally relates to the use of a compound of methoxychlor in the manufacture of a medicament for the treatment of Alzheimer's disease by increasing chaperone protein levels. The chaperone protein of interest is one of the stress proteins, preferably one of the family of thiol protein disulfide oxidoreductase (TPDO), more preferably ERPS7.

The present invention also includes the use of a compound of methoxychlor in the manufacture of a medicament for alleviating symptoms of Alzheimer's disease. The medicament may include a substituted biphenylmethane compound or a pharmaceutical composition of the compound that is chosen due to its ability to increase ERP57.

### Brief Description of the Drawing

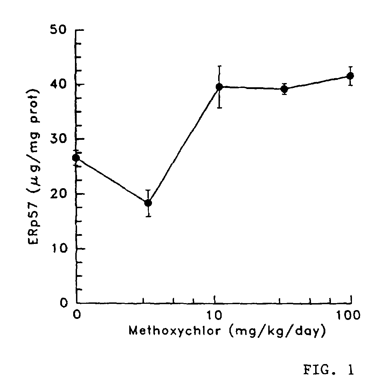

Figure 1 illustrates levels of ERP57 as a function of methoxychlor dosage.

### Detailed Description of the Preferred Embodiment

The present invention relates to use of methoxychlor in the manufacture of a medicament for the treatment of Alzheimer's disease, in particular for increasing levels of the chaperone protein ERP57, or increasing levels of other chaperone proteins, for treating or alleviating a symptom of Alzheimer's disease, or preventing Alzheimer's disease.

### Alzheimer's Disease and β-amyloid

Alzheimer's disease includes the formation of β-amyloid plagues in the brains of subjects afflicted with the disease. The plaques form from aggregates of β-amyloid, which form from cleavage of the amyloid precursor protein and secretion into the intercellular space. β-amyloid freely aggregates in solution in laboratory systems and forms aggregates similar to the plaques formed in Alzheimer's disease.

Amyloid precursor protein, β-amyloid, and various fragments of β-amyloid have been characterized. Known features of amyloid precursor protein and β-amyloid include mammalian genes encoding them, recombinant expression systems (e.g. vectors, plasmids, and the like) for these proteins, methods of producing these proteins, protein sequences and structures, and proteases that cleave the amyloid precursor to β-amyloid.

β-amyloid can be made and/or isolated in a variety of forms. The most prevalent form of β-amyloid in mammalian tissues is β-amyloid 1-42, where the numbering represents the number of amino acids starting at the amino terminus of complete β-amyloid, which has from 38-43 amino acids. The second most prevalent form of β-amyloid in mammalian tissues is β-amyloid 1-38.

β-amyloids have been produced by chemical and/or biotechnical methods, characterized, and shown to have many of the properties of complete β-amyloid. β-amyloid as used herein refers to all of the various forms of β-amyloid, including glycosylated, nonglycosylated, forms of various lengths, and the like.

### Chaperones

Chaperones (also known as chaperone proteins, and including chaperonins and certain heat shock proteins) catalyze folding, formation of tertiary structure, formation of quaternary structure, and/or other processing necessary to make an active protein. As described herein, several chaperones can decline in level with age and can be correlated with age-related diseases and disorders. In particular, tissue levels of one or more of the chaperones BiP, calreticulin, calnexin, ERp72, ERP57, and ERp55 can decrease with age of a mammal, such as a rodent or a human, and can correlate with a disease.

Chaperones include a family known as a thiol:protein disulfide oxidoreductase (TPDO). A TPDO represents a preferred chaperone of the present invention. A preferred TPDO is TPDO-ERP57. TPDO-ERP57 has also been called ERp57, ERp60, ERp61, and GRp58. As used herein, ERP57 refers to any of the common names for this protein, including TPDO-ERP57, ERp57, , ERp60, ERp61, and GRp58 and all naturally occurring variant forms of this protein, including glycosylated and nonglycosylated forms.

Chaperones, such as ERP57, are in general, well studied and/or characterized proteins. Well characterized features of numerous chaperones, such as ERP57, include the genes encoding them in organisms ranging from worms to humans, recombinant expression systems (e.g. vectors, plasmids, and the like) for these proteins, methods of producing these proteins, protein sequences and structures, certain protein substrates, and certain biological functions. Chaperones, such as ERP57, have previously been observed to decrease with the age of a mammal and have been implicated in Alzheimer's disease or formation of β-amyloid plaques.

### A Complex of Chaperone ERP57 and β-amyloid

Although other chaperones may be present in cerebrospinal fluid, only ERP57 has been identified in cerebrospinal fluid. ERP57 levels can decrease relative to normal ERP57 levels in mammalian tissue with age. The term "normal ERP57 levels" as used herein includes the mean concentration of ERP57 that can typically be found in cerebrospinal fluid from a control population. Suitable control populations include, for example, young people, elderly people without Alzheimer's disease, and the like. A level of ERP57 measured in three control populations is about 27 ± 2 ng/ml.

ERP57 can form a complex with β-amyloid. This complex can be isolated from cerebrospinal fluid. ERP57 and β-amyloid are believed to form a strongly bound complex. For instance, immunoblots evidence very little dissociation of the complex. The complex of ERP57 and β-amyloid is believed to be an intermediate in normal processing of amyloid precursor proteins in subjects not suffering from Alzheimer's disease. A decrease in the level of ERP57 relative to normal ERP57 levels in an aging animal can lead to decreased amounts of the complex and increased amounts of free β-amyloid. Increased amounts of free β-amyloid can give rise to increased deposition of amyloid plaques that are associated with Alzheimer's disease.

The complex of ERP57 and β-amyloid can be isolated from cerebrospinal fluid, purified, and characterized by common methods of protein chemistry. For example, the complex can be isolated and/or purified by affinity chromatography with a system having affinity for one or either of ERP57 or β-amyloid. The complex can then be further purified by other forms of chromatography, such as separation on a Sephacryl column and/or a monoQ column. Following isolation and/or purification, the complex can be characterized by employing standard methods such as peptide mapping, sequencing, the PAS reaction (periodic acid-Schiff reaction), and matrix assisted laser desorption ionization-time of flight mass spectrometry (MALDI-TOF).

The complex of ERP57 and β-amyloid can be glycosylated in natural systems. Under most circumstances outside the complex, neither ERP57 nor β-amyloid appear to be glycosylated. Typically the complex is glycosylated, for example, N-glycosylated on an asparagine residue (e.g. asparagine 27) with a complex oligosaccharide.

### Detecting ERP57 Levels

The complex of ERP57 and β-amyloid and the unbound components ERP57 and β-amyloid can be detected by methods known for detecting proteins, sugars, and/or glycosylated proteins. The complex has a molecular weight of about 62 kD on gently or moderately denaturing gel electrophoresis and is recognized by antibodies to either ERP57 or β-amyloid. Polyclonal or monoclonal antibodies recognizing either ERP57 or β-amyloid are known in the art and can be produced by standard methods. Such antibodies can be labeled or otherwise employed in standard immunoassays. Preferred standard immunoassays include ELISA assays, immunoblots, sandwich assays, enhanced chemiluminescence, and the like. Additional methods for detecting the complex or its unbound components include fluorescence polarization assay, standard protein purification by column and affinity chromatography, proteolytic cleavage followed by Edman degradation analysis, and time-of-flight mass spectrometry (e.g. MALDI-TOF).

Methods for detecting ERP57 levels in biological samples can be employed in a method for detecting, determining, examining, diagnosing, screening for, or otherwise assessing a patient's or subject's susceptibility to Alzheimer's disease. The term "susceptibility to Alzheimer's disease" as used herein includes the presence or absence of Alzheimer's disease, of symptoms of this disease, of factors leading to or associated with this disease, of a likelihood of developing this disease in a subject or patient, and the like. "Susceptibility to Alzheimer's disease" can also be defined in terms of a level of ERP57 below that found in a control population. As used herein, factors leading to or associated with this disease include formation of β-amyloid plaques, disorders in processing of β-amyloid precursor protein, and the like.

As used herein biological samples include biological material such as a tissue, cell, or fluid sample from an animal or human in need of being screened for or suspected of being susceptible to or suffering from deposition of β-amyloid plaques, disorders in processing of amyloid precursor protein, Alzheimer's disease, likelihood of developing Alzheimer's disease, and the like. Biological samples can also include laboratory samples from an experimental animal, cell, or culture being examined for their propensity to form β-amyloid plaques or aggregates or to improperly process amyloid precursor protein. Biological sample can further include a system designed to express a chaperone protein, such as ERP57, that can be used to study or monitor levels of a chaperone, such as ERP57, and related effects. The biological sample including, for example, tissues or cells, such as liver, platelets, serum, or skin, can be recovered from a living animal or patient by methods such as needle biopsy, venapuncture, or skin scraping. In one aspect of the invention, the biological sample includes biological material from the central nervous system of a human or an animal. A preferred biological sample includes biological material from cerebrospinal fluid for assessing the susceptibility to Alzheimer's disease in living animals or patients. Cerebrospinal fluid can be recovered by spinal tap, during surgical procedures, or by any of the variety of methods known to those of skill in the art.

### Modulating Levels of a Chaperone

### Decline in ERP57 Levels

Generally the decline in ERP57 levels relative to normal ERP57 levels correlates to the susceptibility to Alzheimer's disease. The level of ERP57 includes the level of free ERP57 in the biological sample, the level of ERP57 bound in complex with β-amyloid, the levels of free ERP57 plus bound ERP57, and preferably total ERP57. A decline in the ERP57 level correlates to an increased susceptibility to Alzheimer's disease. An increased susceptibility, as used herein, refers to it being more probable than not that Alzheimer's disease, at least one symptom of the disease, at least one factor leading to or associated with this disease, likelihood of developing this disease, or the like is present in a patient or subject. The decline in ERP57 can be determined relative to a previous level in a subject.

An increased susceptibility to Alzheimer's disease can also be defined in terms of a level of chaperone protein that is less than that found in a control population. For example, in one embodiment a decline in ERP57 level of at least 35% relative to a normal ERP57 level correlates with a 100% likelihood of developing Alzheimer's disease. In another embodiment, the aggregation of β-amyloid in an animal or human can be detected by determining the level of ERP57 in the biological sample. A decline in ERP57 levels relative to normal ERP57 levels correlates to and indicates an increased susceptibility to an increase in β-amyloid aggregation. By detecting the aggregation of β-amyloid, the formation of β-amyloid plaques is also detected.

The susceptibility to Alzheimer's disease may also be determined by correlating the level of free ERP57, the level of β-amyloid, and the level of ERP57:β-amyloid in complex. A relevant decline in ERP57 levels relative to normal ERP57 levels includes a decline of about 35%, preferably about 50%, more preferably about 65 %. Such a relevant decline correlates to an increased susceptibility to Alzheimer's disease, for example, to factors leading to or associated with Alzheimer's disease, particularly β-amyloid plaque formation; to Alzheimer's disease; to symptoms associated with Alzheimer's disease; to developing Alzheimer's disease; or the like.

The decline in ERP57 levels relative to normal ERP57 levels can be correlated with symptoms characteristic of Alzheimer's disease by comparing ERP57 levels with neuropsychiatric function measurements or MRI. Neuropsychiatric function measurements can be made by conducting, for example, a neuropsychiatric evaluation, a complete history, and a physical examination and evaluating this information based on criteria defined by, for example, DSMIII-R and NinCDS-ADRDA. MRI can be conducted by methods known to those of skill in the art. The decline can also be correlated with ERP57 levels below that of a control population.

Increasing levels of ERP57 therefore can have a beneficial effect on Alzheimer's disease and its related symptoms. Modulating levels of ERP57 could either be accomplished by administering ERP57 directly or by administering a compound that serves to increase the natural production of ERP57. ERP57 levels can be enhanced by administration of a suitable compound, or a pharmaceutical composition including a suitable compound. The present invention utilizes methoxychlor to increase the levels of chaperone protein, such as ERP57. Preferably, methoxychlor is administered in order to increase the level of ERP57.

### Decline in Chaperone Protein Levels

A decline in level of a chaperone protein, relative to normal levels of the chaperone protein can also correlate with a susceptibility to Alzheimer's disease. The level of chaperone protein includes the level of free chaperone protein in the biological sample, the level of chaperone protein bound in complex with β-amyloid, the levels of free chaperone protein plus bound chaperone protein, and preferably total chaperone protein. A decline in the chaperone protein level correlates to an increased susceptibility to Alzheimer's disease.

An increased susceptibility to Alzheimer's disease can be defined in terms of a level of chaperone protein that is less than that found in a control population. For example, in one embodiment a decline in chaperone protein level of at least 35% relative to a normal chaperone protein level correlates with a 100% likelihood of developing Alzheimer's disease. In another embodiment, the aggregation of β-amyloid in an animal or human can be detected by determining the level of chaperone protein in the biological sample. A decline in chaperone protein levels relative to normal chaperone protein levels correlates to and indicates an increased susceptibility to an increase in β-amyloid aggregation. By detecting the aggregation of β-amyloid, the formation of β-amyloid plaques is also detected.

Susceptibility to Alzheimer's disease can also be determined by correlating the level of free chaperone protein, the level of β-amyloid, and the ratio of chaperone protein:β-amyloid in complex. A relevant decline in chaperone protein levels relative to normal chaperone protein levels includes a decline of about 35%, preferably about 50%, more preferably about 65%. Such a relevant decline correlates to an increased susceptibility to Alzheimer's disease, for example, to factors leading to or associated with Alzheimer's disease, particularly β-amyloid plaque formation; to Alzheimer's disease; to symptoms associated with Alzheimer's disease; to developing Alzheimer's disease; or the like.

The decline in chaperone protein levels relative to normal chaperone protein levels can be correlated with symptoms characteristic of Alzheimer's disease by comparing chaperone protein levels with neuropsychiatric function measurements. Neuropsychiatric function measurements can be made by conducting, for example, a neuropsychiatric evaluation, a complete history, and a physical examination and evaluating this information based on criteria defined by, for example, DSMIII-R and NinCDS-ADRDA. The decline can also be correlated with chaperone protein levels below that of a control population.

Increasing levels of chaperone protein therefore can have a beneficial effect on Alzheimer's disease and its related symptoms. Altering levels of chaperone protein could either be accomplished by administering chaperone protein directly or by administering a compound that serves to increase the natural production of chaperone protein. Chaperone protein levels can be enhanced by administration of a suitable compound, or a pharmaceutical composition including a suitable compound. The present invention utilizes methoxychlor to increase the levels of chaperone protein, such as ERP57. Preferably, methoxychlor is administered in order to increase the level of ERP57.

### Increase in Levels of a Chaperone Protein

As mentioned above, an increase in level of a chaperone protein, such as ERP57, correlates to the prevention, treatment, or reduction of symptoms of includes an increase in the level of free chaperone protein, such as ERP57, in the biological sample, an increase in the level of chaperone protein, such as ERP57, bound in complex with β-amyloid, an increase in the level of free chaperone protein, plus bound chaperone protein, and preferably an increase in total level of chaperone protein, such as ERP57.

Prevention, treatment, or reduction of symptoms of Alzheimer's disease, as used herein, results from administration of an "effective amount" of a suitable compound. An "effective amount" is an amount sufficient to prevent, treat, reduce and/or ameliorate the symptoms and/or underlying causes of any of Alzheimer's disease, for example, by increasing a level of a chaperone protein, such as ERP57. In some instances, an "effective amount" is sufficient to eliminate the symptoms of those diseases and, perhaps, overcome the disease itself. In the context of the present invention, the terms "treat" and "therapy" and the like refer to alleviate, slow the progression, prophylaxis, attenuation or cure of existing disease. Prevent, as used herein, refers to putting off, delaying, slowing, inhibiting, or otherwise stopping, reducing, or ameliorating the onset of such brain diseases or disorders. It is preferred that a large enough quantity of the compound be administered at non-toxic levels to provide an effective level of activity within the body of the treated mammal. The methods and compositions may be used with any mammal. Exemplary mammals include, but are not limited to rats, cats, dogs, horses, cows, sheep, pigs, and more preferably humans.

In one embodiment, an increase in level of a chaperone protein, such as ERP57, so that the levels are within 40% of the levels found in a control population correlates with treatment or prevention of Alzheimer's disease. Preferably an increase in a chaperone protein level, such as ERP57 to within 20% of the levels found in a control population correlates with treatment or prevention of Alzheimer's disease. More preferably, an increase in a chaperone protein level, such as ERP57 to within 10% of the level found in a control population correlates with treatment or prevention of Alzheimer's disease.

In another embodiment, raising the level of ERP57 to within 40% of 27 ng/ml correlates with treatment or prevention of Alzheimer's disease. Preferably, raising the level of ERP57 to within 20% of 27 ng/ml correlates with treatment or prevention of Alzheimer's disease. More preferably, raising the level of ERP57 to within 10% of 27 ng/ml correlates with treatment or prevention of Alzheimer's disease.

In yet another embodiment, the aggregation of β-amyloid in an animal or human can be detected by determining the level of ERP57 in the biological sample. An increase in the level of chaperone proteins, such as ERP57 relative to the levels in a person who is susceptible to Alzheimer's disease indicates a decreased susceptibility to β-amyloid aggregation. By detecting the aggregation of β-amyloid, the formation of β-amyloid plaques is also detected. Therefore, this correlates with the treatment or prevention of Alzheimer's disease.

The treatment and prevention of Alzheimer's disease can also be monitored by correlating the level of free chaperone protein, such as ERP57, the level of β-amyloid, and the level of chaperone protein (ERP57):β-amyloid in complex. A relevant increase in a chaperone protein level, such as ERP57, relative to the level of chaperone protein, such as ERP57, seen in patients susceptible to Alzheimer's disease includes an increase of 10%, preferably about 20%, more preferably about 40% compared to levels before treatment. Another measure of the relevant levels of chaperone proteins, such as ERP57, is that they are within 20% of the levels found in a control population. Further, the relevant levels of ERP57 could be characterized as being within 20% of 27 ng/ml. Such a relevant increase correlates to treatment or prevention of Alzheimer's disease, for example, to factors leading to or associated with Alzheimer's disease, particularly β-amyloid plaque formation; to symptoms associated with Alzheimer's disease; to Alzheimer's disease; to developing Alzheimer's disease or the like.

The increase in chaperone proteins, such as ERP57, in a patient who is susceptible to Alzheimer's disease can be correlated with the alleviation of symptoms characteristic of Alzheimer's disease by comparing a level of a chaperone protein, such as ERP57, with neuropsychiatric function measurements. Neuropsychiatric function measurements can be made by conducting, for example, a neuropsychiatric evaluation, a complete history, a physical examination, an MRI, or the like, and then evaluating this information based on criteria defined by, for example, DSMIII-R and NinCDS-ADRDA. The alleviation of symptoms characteristic of Alzheimer's disease can also be correlated with chaperone protein levels below that of a control population.

In some instances, individuals who have a level of a chaperone protein, such as ERP57, of about normal can also be susceptible to Alzheimer's disease. For example, individuals having a normal level of a chaperone protein, such as ERP57, but an apoE₄ allele have an increased susceptibility to Alzheimer's disease. There may be other risk factors for Alzheimer's disease that result in an increased susceptibility to Alzheimer's disease in the presence of a normal level of a chaperone protein, such as ERP57.

### Substituted Biphenylmethane Compounds for Incresing ERP57

### Methoxychlor

The compound used according to the present invention is methoxychlor, 1,1,1-trichloro-2,2-bis-(4-methoxyphenyl)ethane (Formula II below). 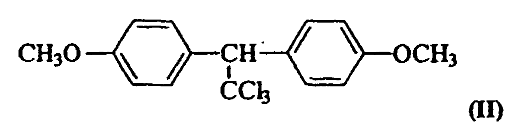 Methoxychlor is a member of the family of organochloride insecticides, which includes methoxychlor analogs. Such methoxychlor analogs include DDT [1,1,1-trichloro-2,2-bis-(4-chlorophenyl)ethane], perthane [1,1-dichloro-2,2-bis-(4-ethylphenyl)ethane], and DFDT [1,1,1-trichloro-2,2-bis-(4-fluorophenyl)ethane]. The organochlorine insecticides have been used since 1939.

Methoxychlor is generally considered safer than DDT because of the lowered level of bioaccumulation. Methoxychlor is considered one of the safest of all insecticides, with a rat oral LD₅₀ of > 6000 mg/kg. It is used widely in the home garden, for control of insect pests of vegetables and fruits, and for the control of bark beetles that are vectors of Dutch elm disease.

### Pharmaceutical Compositions

Increases in the amount of a chaperone protein, such as ERP57 to a therapeutically effective level may be obtained via administration of a pharmaceutical composition including a therapeutically effective dose of a suitable substituted biphenylmethane compound as described above. "Therapeutically effective dose" is intended to mean a dose of the compound that achieves the desired goal of increasing the amount of ERP57 protein. The increase in ERP57 can either be measured relative to the level in the patient before administration or as a goal of reaching a desired level of ERP57. A desired level of ERP57 protein can be characterized as a level that is sufficient to either decrease, or inhibit plaque formation within the brain. The level could also be characterized as one that is sufficient to reduce or inhibit the display of symptoms commonly associated with plaque formation within the brain. The level could also be characterized as one that is sufficient to reduce the susceptibility of a patient to Alzheimer's disease.

The use of the invention is directed to a composition that can be employed to increase the concentration of chaperone proteins, such as ERP57. The composition can include, for example, any pharmaceutically acceptable additive, carrier, or adjuvant that is suitable for administering an agent to a mammal. Preferably, the pharmaceutical composition can be employed in diagnosis, prevention, or treatment of a disease, disorder, or injury that relates to chaperone levels in a patient. Preferably, the composition includes an agent in combination with a pharmaceutical carrier, additive, and/or adjuvant that can promote the transfer of the compound within or throughout the patient.

The composition typically contains a pharmaceutically acceptable carrier mixed with the agent and other components in the pharmaceutical composition. By "pharmaceutically acceptable carrier" is intended a carrier that is conventionally used in the art to facilitate the storage, administration, and/or the healing effect of the agent. A carrier may also reduce any undesirable side effects of the agent. A suitable carrier should be stable, i.e., incapable of reacting with other ingredients in the formulation. It should not produce significant local or systemic adverse effect in recipients at the dosages and concentrations employed for treatment. A pharmaceutically acceptable carrier is one that is suitable for human administration and does not include compounds that are utilized in animal toxicological studies. Such carriers are generally known in the art.

Suitable carriers for this invention include those conventionally used such as albumin, gelatin, collagen, polysaccharide, monosaccharides, polyvinylpyrrolidone, polylactic acid, polyglycolic acid, polymeric amino acids, fixed oils, ethyl oleate, liposomes, glucose, sucrose, lactose, mannose, dextrose, dextran, cellulose, mannitol, sorbitol, polyethylene glycol (PEG), and the like.

Water, saline, aqueous dextrose, and glycols are preferred liquid carriers, particularly (when isotonic) for solutions. The carrier can be selected from various oils, including those of petroleum, animal, vegetable or synthetic origin, for example, peanut oil, soybean oil, mineral oil, sesame oil, and the like. Suitable pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. The compositions can be subjected to conventional pharmaceutical expedients, such as sterilization, and can contain conventional pharmaceutical additives, such as preservatives, stabilizing agents, wetting, or emulsifying agents, salts for adjusting osmotic pressure, buffers, and the like.

Other acceptable components in the composition include, but are not limited to, buffers that enhance isotonicity such as water, saline, phosphate, citrate, succinate, acetic acid, and other organic acids or their salts. Typically, the pharmaceutically acceptable carrier also includes one or more stabilizers, reducing agents, anti-oxidants and/or anti-oxidant chelating agents. The use of buffers, stabilizers, reducing agents, anti-oxidants and chelating agents in the preparation of protein based compositions, particularly pharmaceutical compositions, is well-known in the art. *See,* Wang *et al., "Review of Excipients and pHs for Parenteral Products Used in the United States. "J. Parent. Drug Assn.,* 34(6):452-462 (1980); Wang *et al., "Parenteral Formulations of Proteins and Peptides: Stability and Stabilizers, "J. Parent. Sci. and Tech.,* 42:S4-S26 (Supplement 1988); Lachman, *et al., "Antioxidants and Chelating Agents as Stabilizers in Liquid Dosage Forms-Part 1, "Drug and Cosmetic Industry,* 102(1): 36-38, 40 and 146-148 (1968); Akers, M.J., *"Antioxidants in Pharmaceutical Products, "J. Parent. Sci. and Tech.,* 36(5):222-228 (1988); and Methods in Enzymology, Vol. XXV, Colowick and Kaplan eds., *"Reduction of Disulfide Bonds in Proteins with Dithiothreitol,* " by Konigsberg, pages 185-188.

Suitable buffers include acetate, adipate, benzoate, citrate, lactate, maleate, phosphate, tartarate, borate, tri(hydroxymethyl aminomethane), succinate, glycine, histidine, the salts of various amino acids, or the like, or combinations thereof. *See* Wang (1980) at page 455. Suitable salts and isotonicifiers include sodium chloride, dextrose, mannitol, sucrose, trehalose, or the like. Where the carrier is a liquid, it is preferred that the carrier is hypotonic or isotonic with oral, conjunctival or dermal fluids and have a pH within the range of 4.5-8.5. Where the carrier is in powdered form, it is preferred that the carrier is also within an acceptable non-toxic pH range.

Suitable reducing agents, which maintain the reduction of reduced cysteines, include dithiothreitol (DTT also known as Cleland's reagent) or dithioerythritol at 0.01% to 0.1% wt/wt; acetylcysteine or cysteine at 0.1% to 0.5% (pH 2-3); and thioglycerol at 0.1% to 0.5% (pH 3.5 to 7.0) and glutathione. *See* Akers (1988) at pages 225 to 226. Suitable antioxidants include sodium bisulfite, sodium sulfite, sodium metabisulfite, sodium thiosulfate, sodium formaldehyde sulfoxylate, and ascorbic acid. *See* Akers (1988) at pages 225. Suitable chelating agents, which chelate trace metals to prevent the trace metal catalyzed oxidation of reduced cysteines, include citrate, tartarate, ethylenediaminetetraacetic acid (EDTA) in its disodium, tetrasodium, and calcium disodium salts, and diethylenetriamine pentaacetic acid (DTPA). *See e.g.,* Wang (1980) at pages 457-458 and 460-461, and Akers (1988) at pages 224-227.

The composition can include one or more preservatives such as phenol, cresol, paraaminobenzoic acid, BDSA, sorbitrate, chlorhexidine, benzalkonium chloride, or the like. Suitable stabilizers include carbohydrates such as threlose or glycerol. The composition can include a stabilizer such as one or more of microcrystalline cellulose, magnesium stearate, mannitol, sucrose to stabilize, for example, the physical form of the composition; and one or more of glycine, arginine, hydrolyzed collagen, or protease inhibitors to stabilize, for example, the chemical structure of the composition. Suitable suspending agents include carboxymethyl cellulose, hydroxypropyl methylcellulose, hyaluronic acid, alginate, chondroitin sulfate, dextran, maltodextrin, dextran sulfate, or the like. The composition can include an emulsifier such as polysorbate 20, polysorbate 80, pluronic, triolein, soybean oil, lecithins, squalene and squalanes, sorbitan treioleate, or the like. The composition can include an antimicrobial such as phenylethyl alcohol, phenol, cresol, benzalkonium chloride, phenoxyethanol, chlorhexidine, thimerosol, or the like. Suitable thickeners include natural polysaccharides such as mannans, arabinans, alginate, hyaluronic acid, dextrose, or the like; and synthetic ones like the PEG hydrogels of low molecular weight and aforementioned suspending agents.

The composition can include an adjuvant such as cetyl trimethyl ammonium bromide, BDSA, cholate, deoxycholate, polysorbate 20 and 80, fusidic acid, or the like, and in the case of DNA delivery, preferably, a cationic lipid. Suitable sugars include glycerol, threose, glucose, galactose and mannitol, sorbitol. A suitable protein is human serum albumin.

Preferred compositions include one or more of a solubility enhancing additive, preferably a cyclodextrin; a hydrophilic additive, preferably a mono or oligosaccharide; an absorption promoting additives, preferably a cholate, a deoxycholate, a fusidic acid, or a chitosan; a cationic surfactant, preferably a cetyl trimethyl ammonium bromide; a viscosity enhancing additive, preferably to promote residence time of the composition at the site of administration, preferably a carboxymethyl cellulose, a maltodextrin, an alginic acid, a hyaluronic acid, or a chondroitin sulfate; or a sustained release matrix, preferably a polyanhydride, a polyorthoester, a hydrogel, a particulate slow release depo system, preferably a polylactide co-glycolides (PLG), a depo foam, a starch microsphere, or a cellulose derived buccal system; a lipid based carrier, preferably an emulsion, a liposome, a niosomes, or a micelles. The composition can include a bilayer destabilizing additive, preferably a phosphatidyl ethanolamine; a fusogenic additive, preferably a cholesterol hemisuccinate.

Other preferred compositions for sublingual administration include employing a bioadhesive to retain the agent sublingually; a spray, paint, or swab applied to the tongue; retaining a slow dissolving pill or lozenge under the tongue; or the like. Administration of agent through the skin can be accomplished by a variety of methods known to those of skill in the art for transdermal delivery, including a transdermal patch, an ointment, an iontophoretic patch or device, and the like. Other preferred methods for transdermal administration include a bioadhesive to retain the agent on or in the skin; a spray, paint, cosmetic, or swab applied to the skin; or the like.

These lists of carriers and additives is by no means complete and a worker skilled in the art can choose excipients from the GRAS (generally regarded as safe) list of chemicals allowed in the pharmaceutical preparations and those that are currently allowed in topical and parenteral formulations.

For the purposes of this invention, the pharmaceutical composition including suitable compound can be formulated in a unit dosage and in a form such as a solution, suspension, or emulsion. The agent may be administered to the nasal cavity as a powder, a granule, a solution, a cream, a spray (e.g., an aerosol), a gel, an ointment, an infusion, an injection, a drop, or sustained release composition, such as a polymer disk. For buccal administration, the compositions can take the form of tablets or lozenges formulated in a conventional manner. For administration to the eye or other external tissues, e.g., mouth and skin, the compositions can be applied to the infected part of the body of the patient as a topical ointment or cream. The compounds can be presented in an ointment, for instance with a water-soluble ointment base, or in a cream, for instance with an oil in water cream base. For conjunctival applications, the compound can be administered in biodegradable or non-degradable ocular inserts. The drug may be released by matrix erosion or passively through a pore as in ethylene-vinylacetate polymer inserts. For other mucosal administrations such as sublingual, powder discs may be placed under the tongue and active delivery systems may for in situ by slow hydration as in the formulation of liposomes from dried lipid mixtures or pro-liposomes.

Other preferred forms of compositions for administration include a suspension of a particulate, such as an emulsion, a liposome, an insert that releases the agent slowly, and the like. The powder or granular forms of the pharmaceutical composition may be combined with a solution and with a diluting, dispersing or surface active agent. Additional preferred compositions for administration include a bioadhesive to retain the agent at the site of administration; a spray, paint, or swab applied to the mucosa or epithelium; a slow dissolving pill or lozenge, or the like. The composition can also be in the form of lyophilized powder, which can be converted into solution, suspension, or emulsion before administration. The pharmaceutical composition having agent is preferably sterilized by membrane filtration and is stored in unit-dose or multidose containers such as sealed vials or ampoules.

The method for formulating a pharmaceutical composition is generally known in the art. A thorough discussion of formulation and selection of pharmaceutically acceptable carriers, stabilizers, and osomolytes can be found in *Remington's Pharmaceutical Sciences* (18^{th} ed.; Mack Publishing Company, Eaton, Pennsylvania, 1990),

A suitable compound for the use of the present invention can also be formulated in a sustained-release form to prolong the presence of the pharmaceutically active agent in the treated mammal, generally for longer than one day. Many methods of preparation of a sustained-release formulation are known in the art and are disclosed in *Remington's Pharmaceutical Sciences* (18^{th} ed.; Mack Publishing Company, Eaton, Pennsylvania, 1990).

Generally, the agent can be entrapped in semipermeable matrices of solid hydrophobic polymers. The matrices can be shaped into films or microcapsules. Examples of such matrices include, but are not limited to, polyesters, copolymers of L-glutamic acid and gamma ethyl-L-glutamate (Sidman *et al.* (1983) *Biopolymers* 22: 547-556), polylactides (U.S. Patent No. 3,773,919 and EP 58,481), polylactate polyglycolate (PLGA) such as polylactide-co-glycolide (see, for example, U.S. Patent Nos. 4,767,628 and 5,654,008), hydrogels (see, for example, Langer *et al.* (1981) *J. Biomed. Mater. Res.* 15: 167-277; Langer (1982) *Chem. Tech.* 12: 98-105), non-degradable ethylene-vinyl acetate (e.g. ethylene vinyl acetate disks and poly(ethylene-co-vinyl acetate)), degradable lactic acid-glycolic acid copolymers such as the Lupron Depot, poly-D-(-)-3-hydroxybutyric acid (EP 133,988), hyaluronic acid gels (see, for example, U.S. Patent 4,636,524), alginic acid suspensions, and the like.

Suitable microcapsules can also include hydroxymethylcellulose or gelatin-microcapsules and polymethyl methacrylate microcapsules prepared by coacervation techniques or by interfacial polymerization. See the copending application entitled *"Method for Producing Sustained-release Formulations,"* U.S. Patent Application Serial No. 09/187,780, filed November 6, 1998, wherein a agent is encapsulated in PLGA microspheres. In addition, microemulsions or colloidal drug delivery systems such as liposomes and albumin microspheres, may also be used. See *Remington's Pharmaceutical Sciences* (18^{th} ed.; Mack Publishing Company Co., Eaton, Pennsylvania, 1990). Other preferred sustained release compositions employ a bioadhesive to retain the agent at the site of administration.

### Administering the Compound

A suitable medicament of the present invention is typically administered in a dose sufficient to provide a therapeutically effective level. Levels of ERP57 can be determined via the methods given above in the section "Detecting ERP57 Levels."

The dose of the compound can be determined by correlating the amount administered to the resulting increase in the desired chaperone protein, such as ERP57, a decrease in the susceptibility to Alzheimer's disease, a decrease in the β-amyloid plaque formation, or a decrease in the amount of free β-amyloid, as enumerated above. The dose of the compound can be 0.1, 0.2, 0.5, 1, 2, 5, 10, 20, 50, 100, 250, 500, 1000, 2000, or 3,000 mg/kg/day.

It is recognized that the total amount of the compound administered as a unit dose to a particular tissue will depend upon the type of pharmaceutical composition being administered, that is whether the composition is in the form of, for example, a solution, a suspension, an emulsion, or a sustained-release formulation. For example, where the pharmaceutical composition including a therapeutically effective amount of a suitable compound is a sustained-release formulation, the compound is administered at a higher concentration.

It should be apparent to a person skilled in the art that variations may be acceptable with respect to the therapeutically effective dose and frequency of the administration of a suitable compound. The amount of the suitable compound administered will be inversely correlated with the frequency of administration. Hence, an increase in the concentration of the suitable compound in a single administered dose, or an increase in the mean residence time in the case of a sustained release form of the compound, generally will be coupled with a decrease in the frequency of administration.

It is appreciated by those of skill in the art that the actual dose of a suitable compound will depend on a variety of factors that may be specific to the subject undergoing dosing. These factors should be taken into consideration when determining the therapeutically effective dose of the compound and frequency of its administration. For example, the effective dose can depend on the species, age, weight, or general health of the subject; the severity of the disease or disorder; the size and location of the portion of the brain in which an effective amount of compound must be achieved; the frequency and duration of dosing; the type of formulation administered; the characteristics, such as lipophilicity, of the compound and composition; the nature of the agent and its receptors, if any; and the like. Generally, a higher dosage is preferred if the disease or disorder is more severe.

Some minor degree of experimentation may be required to determine the most effective dose and frequency of dose administration, this being well within the capability of one skilled in the art once apprised of the present disclosure.

### Intermittent Dosing

The pharmaceutical composition including the therapeutically effective dose of the compound can be administered intermittently. By "intermittent administration" is intended administration of a therapeutically effective dose of the composition, followed by a time period of discontinuance, which is then followed by another administration of a therapeutically effective dose, and so forth. Administration of the therapeutically effective dose may be achieved in a continuous manner, as for example with a sustained-release formulation, or it may be achieved according to a desired daily dosage regimen, as for example with one, two, three or more administrations per day. By "time period of discontinuance" is intended a discontinuing of the continuous sustained-released or daily administration of the composition. The time period of discontinuance may be longer or shorter than the period of continuous sustained-release or daily administration. During the time period of discontinuance, the compound level in the relevant tissue is substantially below the maximum level obtained during the treatment. The preferred length of the discontinuance period depends on the concentration of the effective dose and the form of the composition used. The discontinuance period can be at least 2 days, preferably is at least 4 days, more preferably is at least 1 week and generally does not exceed a period of 4 weeks. When a sustained-release formulation is used, the discontinuance period must be extended to account for the greater residence time of the composition at the site of injury. Alternatively, the frequency of administration of the effective dose of the sustained-release formulation can be decreased accordingly. An intermittent schedule of administration of the composition can continue until the desired therapeutic effect, and ultimately treatment of the disease or disorder, is achieved.

Intermittent administration of the therapeutically effective dose of the composition may be cyclic. By "cyclic" is intended intermittent administration accompanied by breaks in the administration, with cycles ranging from about 1 month to about 2, 3, 4, 5, or 6 months, more preferably about 3 months to about 6 months. For example, the administration schedule might be intermittent administration of the effective dose of the composition, wherein a single short-term dose is given once per week for 4 weeks, followed by a break in intermittent administration for a period of 3 months, followed by intermittent administration by administration of a single short-term dose given once per week for 4 weeks, followed by a break in intermittent administration for a period of 3 months, and so forth. As another example, a single short-term dose may be given once per week for 2 weeks, followed by a break in intermittent administration for a period of 1 month, followed by a single short-term dose given once per week for 2 weeks, followed by a break in intermittent administration for a period of 1 month, and so forth. A cyclic intermittent schedule of administration of agent to subject may continue until the desired therapeutic effect, and ultimately treatment of the disorder or disease, is achieved.

The present invention may be better understood with reference to the following examples.

### EXAMPLES

### Example 1 - Effect of Methoxychlor on ERP57 Concentration in Rat Livers

The purpose of this experiment was to determine the effect of administering varying levels of methoxychlor for 14 days on the levels of the microsomal protein ERP57 in mammalian tissues.

### Materials and Methods

Methoxychlor, potassium chloride, and Tris were purchased from Sigma Chemicals (St. Louis, MO). The BCA (Bicinchoninic Acid) Assay kits were purchased from Pierce Chemical Company (Rockford, IL). Agarose was purchased from Boehringer Mannheim (Indianapolis, IN). Polyclonal antibodies to the ERP57 were developed in laying hens as previously described (Srivastava *et al.* 1991, *J. Biol. Chem.* **266**: 20337-20344; Chen *et al.* 1996, *Biochemistry* **25**: 8299-8306).

The animals used in these studies were 150-200 g fed, male, CD rats from Harlan Laboratories. They received either methoxychlor in corn oil or the vehicle alone (0.8 ml) by gavage. The animals were euthanized with CO₂ and their livers were removed, weighed and minced in cold KCl-Tris ( 150 mM-50 mM; pH 7.2). The individual livers were separately homogenized in KCl-Tris (4 ml/g liver) and the homogenates were centrifuged for 20 minutes at 10,000 x g. The supernatants were centrifuged for 1 hour at 100,000 x g. The pellets were washed and resuspended in KCl-Tris and recentrifuged for 1 hour at 100,000 x g. The final microsomal pellets were suspended in KCl-Tris (0.5 ml/g liver). The microsomal suspensions were aliquoted and stored at -80°C until analyzed.

The concentrations of total microsomal protein were then determined via the BCA assay. Statistical significance was estimated by the Student t-test. A two tailed p-value of less than 0.05 was taken as significant.

The concentration of ERP57 in the samples was determined with immunoelectrophoresis. Protein sodium dodecyl sulfate (SDS)-polyacrylamide gel electrophoresis was performed by the method of Laemmli (Laemmli 1970, *Nature* **227**: 680-685) with a 3% stacking gel and a 12% running gel. The proteins were transferred from the gel to PVDF membranes in CAPS buffer as described in Towbin *et al.* (Towbin *et al.* 1978, *Proc. Natl. Acad. Sci.* **76**: 4350-4354). The membranes were treated with antibody followed by either goat anti-chicken Igy or anti-rabbit Igg conjugated to alkaline phosphatase as appropriate.

The indicator dye was a combination of nitroblue tetrazolium and 5-bromo-4-chloro-3-indoyl phosphate from Bio Rad (Richmond, CA). The membranes were laminated between cellulose sheets. The densities of the stained bands were determined on a flat bed scanner from UMAX (Taiwan, China). The scans were analyzed using NIH Image (version 1.54) on a PowerPC (Apple Computer). The blank for each band was determined from the adjacent, unreacted area in the lane. In control studies, it was found that the immunoassays were linear with respect to the concentrations of the respective proteins and that the band intensities were stable indefinitely after the membranes had been laminated.

### Results

Figure 1 shows the effect of methoxychlor administration on the levels of ERP57 in the rat liver. The concentration of ERP57 was normalized by the total microsomal concentration of protein in the liver sample. As seen there, the relative concentration of ERP57 exhibits a significant increase at the higher dosages, 10, 50, and 100 mg/kg/day.

### Discussion

The results show that the administration of methoxychlor to a subject increases the concentration of ERP57. Because of its association with Alzheimer's disease, an increase of ERP57 concentration could be considered in creating a medicament to treat, or prevent, Alzheimer's disease.

It should be noted that, as used in this specification and the appended claims, the singular forms "a", "an", and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains.

## Claims

1. The use of a compound of formula II; 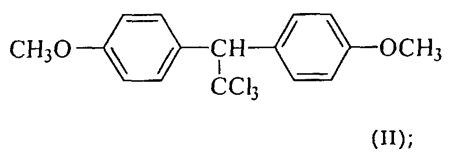 in the manufacture of a medicament for the treatment of Alzheimer's disease.

2. The use of claim 1, wherein the medicament modulates the level of a chaperone protein and the chaperone protein is one or more of BiP, calreticulin, calnexin, ERp72, ERP57, or ERp55.

3. The use of claim 2, wherein the chaperone protein is ERP57.

4. The use of claim 2, wherein the level of the chaperone protein is increased.

5. The use of claim 4, wherein the level of the chaperone protein is increased to within 40% of the level found in a control population.

6. The use of claim 4, wherein the level of the chaperone protein is increased to within 20% of the level found in a control population.

7. The use of claim 4, wherein the level of the chaperone protein is increased to within 10% of the level found in a control population.

8. The use of claim 3, wherein the level of ERP57 is increased to within 40% of 27 ng/ml.

9. The use of claim 3, wherein the level of ERP57 is increased to within 20% of 27 ng/ml.

10. The use of claim 3, wherein the level of ERP57 is increased to within 10% of 27 ng/ml.

11. The use of claim 1, wherein the compound is to be administered at a dosage of 0.1, 0.2, 0.5, 1, 2, 5, 10, 20, 50, 100, 250, 500, 1000, 2000, or 3000 mg/kg/day.

12. The use of claim 1, wherein the compound is to be administered at a dosage of 10, 50 or 100 mg/kg/day.

13. The use of a compound of the formula II; 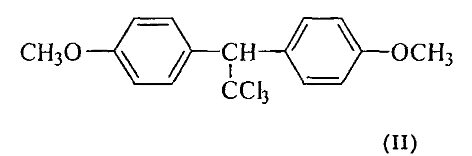 for the manufacture of a medicament for alleviating a symptom of Alzheimer's disease.

14. The use of claim 13, wherein the compound is to be administered at a dosage in the range from 0.1 mg/kg/day to 3000 mg/kg/day.

15. The use of claim 13, wherein the compound is to be administered at a dosage in the range from 0.1 mg/kg/day to 500 mg/kg/day.

16. The use of claim 13, wherein the compound is to be administered at a dosage in the range from 10 mg/kg/day to 100 mg/kg/day.

## Patentansprüche

1. Verwendung einer Verbindung mit Formel II 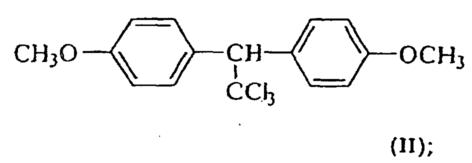 zur Herstellung eines Arzneimittels zur Behandlung von Alzheimer-Krankheit.

2. Verwendung nach Anspruch 1, **dadurch gekennzeichnet, daß** das Arzneimittel den Spiegel eines Chaperon-Proteins moduliert und das Chaperon-Protein eines oder mehrere aus BiP, Calreticulin, Calnexin, ERp72, ERP57 oder ERp55 ist.

3. Verwendung nach Anspruch 2, **dadurch gekennzeichnet, daß** das Chaperon-Protein ERP57 ist.

4. Verwendung nach Anspruch 2, **dadurch gekennzeichnet, daß** der Spiegel des Chaperon-Proteins erhöht wird.

5. Verwendung nach Anspruch 4, **dadurch gekennzeichnet, daß** der Spiegel des Chaperon-Proteins auf innerhalb von 40% des in einer Kontrollpopulation gefundenen Spiegels erhöht wird.

6. Verwendung nach Anspruch 4, **dadurch gekennzeichnet, daß** der Spiegel des Chaperon-Proteins auf innerhalb von 20% des in einer Kontrollpopulation gefundenen Spiegels erhöht wird.

7. Verwendung nach Anspruch 4, **dadurch gekennzeichnet, daß** der Spiegel des Chaperon-Proteins auf innerhalb von 10% des in einer Kontrollpopulation gefundenen Spiegels erhöht wird.

8. Verwendung nach Anspruch 3, **dadurch gekennzeichnet, daß** der Spiegel von ERP57 auf innerhalb von 40% von 27 ng/ml erhöht wird.

9. Verwendung nach Anspruch 3, **dadurch gekennzeichnet, daß** der Spiegel von ERP57 auf innerhalb von 20% von 27 ng/ml erhöht wird.

10. Verwendung nach Anspruch 3, **dadurch gekennzeichnet, daß** der Spiegel von ERP57 auf innerhalb von 10% von 27 ng/ml erhöht wird.

11. Verwendung nach Anspruch 1, **dadurch gekennzeichnet, daß** die Verbindung in einer Dosierung von 0,1, 0,2, 0,5, 1, 2, 5, 10, 20, 50, 100, 250, 500, 1000, 2000 oder 3000 mg/kg/Tag zu verabreichen ist.

12. Verwendung nach Anspruch 1, **dadurch gekennzeichnet, daß** die Verbindung in einer Dosierung von 10, 50 oder 100 mg/kg/Tag zu verabreichen ist.

13. Verwendung einer Verbindung der Formel II 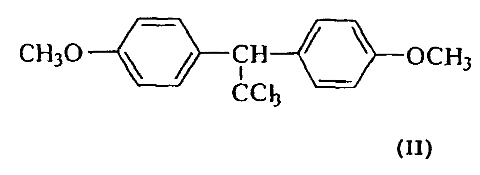 zur Herstellung eines Arzneimittels zur Linderung eines Symptoms der Alzheimer-Krankheit.

14. Verwendung nach Anspruch 13, **dadurch gekennzeichnet, daß** die Verbindung in einer Dosierung im Bereich von 0,1 mg/kg/Tag bis 3.000 mg/kg/Tag zu verabreichen ist.

15. Verwendung nach Anspruch 13, **dadurch gekennzeichnet, daß** die Verbindung in einer Dosierung im Bereich von 0,1 mg/kg/Tag bis 500 mg/kg/Tag zu verabreichen ist.

16. Verwendung nach Anspruch 13, **dadurch gekennzeichnet, daß** die Verbindung in einer Dosierung im Bereich von 10 mg/kg/Tag bis 100 mg/kg/Tag zu verabreichen ist.

## Revendications

1. Utilisation d'un composé de formule II : 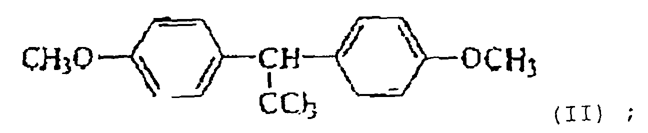 dans la fabrication d'un médicament destiné au traitement de la maladie d'Alzheimer.

2. Utilisation selon la revendication 1, dans laquelle le médicament module le taux d'une protéine chaperon, la protéine chaperon étant l'un ou plusieurs des composés suivants : BiP, calréticuline, calnexine, ERp72, ERp57 ou ERp55.

3. Utilisation selon la revendication 2, dans laquelle la protéine chaperon est ERp57.

4. Utilisation selon la revendication 2, dans laquelle le taux de protéine chaperon est augmenté.

5. Utilisation selon la revendication 4, dans laquelle le taux de la protéine chaperon est augmenté jusqu'à 40% du taux trouvé chez une population témoin.

6. Utilisation selon la revendication 4, dans laquelle le taux de la protéine chaperon est augmenté jusqu'à 20% du taux trouvé chez une population témoin.

7. Utilisation selon la revendication 4, dans laquelle le taux de la protéine chaperon est augmenté jusqu'à 10 % du taux trouvé chez une population témoin.

8. Utilisation selon la revendication 3, dans laquelle le taux de ERp57 est augmenté jusqu'à 40 % de 27 ng/ml.

9. Utilisation selon la revendication 3, dans laquelle le taux de ERp57 est augmenté jusqu'à 20 % de 27 ng/ml.

10. Utilisation selon la revendication 3, dans laquelle le taux de ERp57 est augmenté jusqu'à 10 % de 27 ng/ml.

11. Utilisation selon la revendication 1, dans laquelle le composé doit être administré à une dose de 0,1, 0,2, 0,5, 1, 2, 5, 10, 20, 50, 100, 250, 500, 1000, 2000 ou 3000 mg/kg/jour.

12. Utilisation selon la revendication 1, dans laquelle le composé doit être administré à une dose de 10, 50 ou 100 mg/kg/jour.

13. Utilisation d'un composé de formule II : 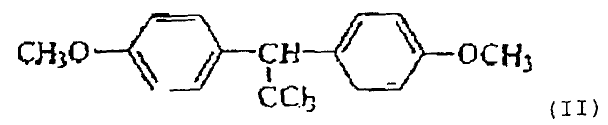 dans la fabrication d'un médicament destiné à soulager un symptôme de la maladie d'Alzheimer.

14. Utilisation selon la revendication 13, dans laquelle le composé doit être administré à une dose comprise dans l'intervalle allant de 0,1 mg/kg/jour à 3000 mg/kg/jour.

15. Utilisation selon la revendication 13, dans laquelle le composé doit être administré à une dose comprise dans l'intervalle allant de 0,1 mg/kg/jour à 500 mg/kg/jour.

16. Utilisation selon la revendication 13, dans laquelle le composé doit être administré à une dose comprise dans l'intervalle allant de 10 mg/kg/jour à 100 mg/kg/jour.
